# EUROPEAN PATENT APPLICATION

(11) **EP 3 827 809 A1**
(43) Date of publication of application: **02.06.2021**
(21) Application number: 19841317.1
(22) Date of filing: 10.07.2019
(51) Int. Cl.: A61K 8/85, A61K 8/02, A61Q 1/00, A61Q 19/00, B32B 27/36

(54) **SKIN ADHESIVE FILM, AND TRANSFER SHEET**

(30) Priority: 25.07.2018 JP 2018139459
(71) Applicant: Toppan Printing Co., Ltd., Tokyo 110-0016 (JP)
(72) Inventor: WATANABE Manabu, Tokyo 110-0016 (JP); HAYASAKA Yuichiro, Tokyo 110-0016 (JP)
(74) Representative: TBK
(86) International application number: PCT/JP2019/027346
(87) International publication number: WO 2020/022064

(57) **Abstract**

A skin-adhesive film comprises a polylactic acid layer. The polylactic acid layer has a thickness of 10 nm or more and 5000 nm or less and contains poly-DL-lactic acid. The poly-DL-lactic acid includes L-lactic acid having a content ratio of 50% or higher and 90% or lower, and the weight-average molecular weight of the poly-DL-lactic acid is 100,000 or higher.

## Description

### [Technical Field]

The present invention relates to a skin-adhesive film, and a transfer sheet including the skin-adhesive film.

### [Background Art]

It has been reported that a thin film having a thickness of several nm to several µm is useful as a skin-adhesive film as an aid to skin care and make-up. As an example of such a skin-adhesive film, PTL 1 discloses a polylactic acid thin film carrying sodium hyaluronate.

### [Citation List]

### [Patent Literature]

[PTL 1] WO 2014/058060

### [Summary of the Invention]

### [Technical Problem]

Melt extrusion and solvent casting are generally known for forming a thin film. Melt extrusion is a method in which a molten material is extruded to form a thin film. Solvent casting is a method in which a thin film is formed from a coating solution prepared by dissolving the material in a solvent, and then the solvent is evaporated. Melt extrusion enables quick and inexpensive film production, but it is difficult to obtain a high yield particularly when forming a thin film. Therefore, solvent casting is more suited to formation of the above-described thin skin-adhesive film.

In an Example of PTL 1, a polylactic acid thin film is formed by dissolving polylactic acid in dichloromethane, applying this solution to a substrate to form a coating film, and then drying the coating film. Further, it has been reported in research conducted by universities and the like that halogen-based solvents such as dichloromethane and chloroform are used as the solvent for forming a polylactic acid thin film. Although halogen-based solvents dissolve the solute well, it cannot be said that they are very safe for human body. Since it is difficult to completely remove the solvent component from the thin film formed by solvent casting, it is not preferable to use a halogen-based solvent as the solvent for forming a skin-adhesive film.

In order to use a solvent that is safer than halogen-based solvents, a skin-adhesive film may be formed by a material that can be dissolved in a solvent that does not dissolve the solute as much as a halogen-based solvent. However, simply forming a skin-adhesive film from a highly soluble material results in a decrease in the durability of the skin-adhesive film to various cosmetics used on the skin. That is, if the skin-adhesive film contacts cosmetics, the skin-adhesive film may be dissolved or become torn. Thus, the durability of the skin-adhesive film is decreased.

The present invention aims to provide a skin-adhesive film that can be manufactured using a solvent that is safer than a halogen-based solvent and is capable of suppressing decrease in the resistance to cosmetics, and a transfer sheet including the skin-adhesive film.

### [Solution to Problem]

A skin-adhesive film for solving the above problems is a skin-adhesive film comprising a polylactic acid layer, wherein the polylactic acid layer has a thickness of 10 nm or more and 5000 nm or less, the polylactic acid layer contains poly-DL-lactic acid, the poly-DL-lactic acid includes L-lactic acid having a content ratio of 50% or more and 90% or less, and the poly-DL-lactic acid has a weight-average molecular weight of 100,000 or more.

According to the above configuration, since the poly-DL-lactic acid includes L-lactic acid having a content ratio of 50% or more and 90% or less, the polylactic acid layer can be formed without using a halogen solvent as the solvent used to manufacture the polylactic acid layer, but using an ester solvent which is safer than halogen solvents. Since the weight-average molecular weight of the poly-DL-lactic acid is 100,000 or more, the resistance of the polylactic acid layer to cosmetics can be improved.

In the above configuration, the weight-average molecular weight of the poly-DL-lactic acid may be less than 800,000.

According to this configuration, since the weight-average molecular weight of the poly-DL-lactic acid is 100,000 or more and less than 800,000 which is suitable for mass production of poly-DL-lactic acid, both the improvement in resistance as described above and improvement in the productivity of the skin-adhesive film can be achieved.

In the above configuration, the skin-adhesive film may further comprise a water-soluble resin layer that is poorly soluble in an ester-based solvent that dissolves poly-DL-lactic acid.

According to the above configuration, the resistance of the skin-adhesive film against the cosmetics and the film durability are improved.

In the above configuration, the skin-adhesive film may further comprise a dodecane-soluble resin layer that is poorly soluble in an ester-based solvent that dissolves poly-DL-lactic acid.

According to the above configuration, the resistance of the skin-adhesive film against the cosmetics and the film durability are improved. In addition, the resistance of the skin-adhesive film to water can also be improved.

In the above configuration, the skin-adhesive film may further comprise a water-soluble resin layer that is poorly soluble in an ester-based solvent that dissolves poly-DL-lactic acid and a dodecane-soluble resin layer that is poorly soluble in the ester-based solvent, and the polylactic acid layer may be sandwiched between the water-soluble resin layer and the dodecane-soluble resin layer.

According to this configuration, a resistance of the skin-adhesive film to a variety of cosmetics is enhanced. Thus, the versatility of the skin-adhesive film is increased.

In the above configuration, the polylactic acid layer may contain water-soluble resin particles, and the water-soluble resin particles may be exposed at a surface of the polylactic acid layer that comes into contact with the water-soluble resin layer.

According to this configuration, the adhesion between the polylactic acid layer and the water-soluble resin layer is improved.

In the above configuration, the polylactic acid layer may contain dodecane-soluble resin particles, and the dodecane-soluble resin particles may be exposed at a surface of the polylactic acid layer that comes into contact with the dodecane-soluble resin layer.

According to this configuration, the adhesion between the polylactic acid layer and the dodecane-soluble resin layer can be improved.

A transfer sheet for solving the above problems comprises: the above-described skin-adhesive film; and a support substrate configured to support the skin-adhesive film and be peelable off from the skin-adhesive film.

According to the above configuration, it is possible to handle a very thin skin-adhesive film in a state where it is supported by a support substrate. Also, it is possible to attach only the skin-adhesive film by peeling the support substrate after the film is attached to the skin. Therefore, the skin-adhesive film can be handled more easily.

### [Effect of the Invention]

According to the present invention, a skin-adhesive film can be manufactured using a solvent that is safer than a halogen-based solvent, and a decrease in its resistance to cosmetics can be suppressed.

### [Brief Description of the Drawings]

Fig. 1 shows the cross-sectional structure of the skin-adhesive film in a first mode with regard to an embodiment of a skin-adhesive film.
Fig. 2 shows the cross-sectional structure of the skin-adhesive film in a second mode.
Fig. 3 shows the cross-sectional structure of the skin-adhesive film in a third mode.
Fig. 4 shows the cross-sectional structure of the skin-adhesive film in a fourth mode.
Fig. 5 shows the cross-sectional structure of the skin-adhesive film in a fifth mode.
Fig. 6 shows the cross-sectional structure of a transfer sheet according to an embodiment.
Fig. 7 shows how the skin-adhesive film including a transfer sheet according to an embodiment is attached, and shows a skin to which a cosmetic has been applied.
Fig. 8 shows how the skin-adhesive film including a transfer sheet according to an embodiment is attached, and shows the step of placing the transfer sheet on the skin.
Fig. 9 shows how the skin-adhesive film including a transfer sheet according to an embodiment is attached, and it shows the skin to which the film has been attached.

### [Description of the Embodiments]

Embodiments of a skin-adhesive film and a transfer sheet will be described with reference to the drawings. The skin-adhesive film according to the present embodiment is attached to the skin for cosmetic purposes such as for skin care and makeup. The skin-adhesive film may be attached over cosmetics applied to the skin, or may be attached to the skin to which no makeup has been applied. Alternatively, the cosmetics may be applied over the film after attaching the film to the skin.

### [Structure of Skin-adhesive Film and Transfer Sheet]

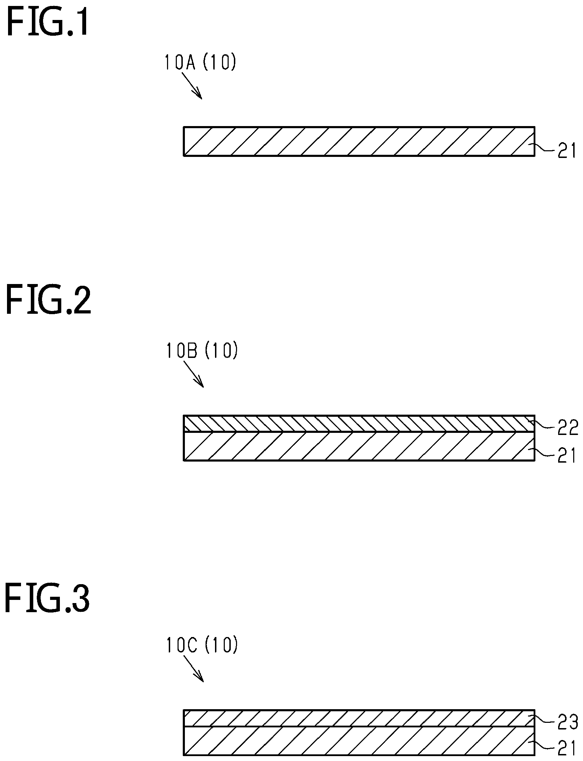

With reference to Figs. 1 to 5, five modes of the skin-adhesive film 10 will be described.

Fig. 1 shows the structure of the skin-adhesive film 10A according to a first mode. The skin-adhesive film 10A includes a polylactic acid layer 21 containing polylactic acid as a main component. The skin-adhesive film 10A in the first mode is solely composed of the polylactic acid layer 21.

Fig. 2 shows the structure of the skin-adhesive film 10B according to a second mode. The skin-adhesive film 10B includes a water-soluble resin layer 22 in addition to the polylactic acid layer 21. The water-soluble resin layer 22 covers one of the two faces of the polylactic acid layer 21. The water-soluble resin layer 22 contains a water-soluble resin as a main component. The water-soluble resin layer 22 has a function of increasing the resistance of the skin-adhesive film 10B to cosmetics.

The skin-adhesive film 10B of the second mode has a two-layer structure including the polylactic acid layer 21 and the water-soluble resin layer 22. The skin-adhesive film 10B has a layer including an attachment surface which is a surface comes in contact with the skin. The layer may be either the polylactic acid layer 21 or the water-soluble resin layer 22. The resistance of the water-soluble resin layer 22 to some cosmetics is higher than that of the polylactic acid layer 21. The skin-adhesive film 10B has the attachment surface and a surface facing to the attachment surface. Thus, a surface which comes into contact with cosmetics is preferably a surface having the water-soluble resin layer 22.

When the water-soluble resin layer 22 has the attachment surface and there is moisture on the skin surface, the moisture softens the water-soluble resin layer 22 and helps the skin-adhesive film 10B to conform to the skin. Accordingly, adhesion between the skin and the skin-adhesive film 10B is enhanced.

Fig. 3 shows the structure of the skin-adhesive film 10C according to a third mode. The skin-adhesive film 10C includes a dodecane-soluble resin layer 23 in addition to the polylactic acid layer 21. The dodecane-soluble resin layer 23 covers one of the two surfaces of the polylactic acid layer 21. The dodecane-soluble resin layer 23 contains, as a main component, a resin that is soluble in dodecane which is a hydrocarbon solvent. The dodecane-soluble resin layer 23 has a function of enhancing the resistance of the skin-adhesive film 10C to cosmetics as well as the resistance of the film 10C to water.

The skin-adhesive film 10C in the third mode has a two-layer structure including the polylactic acid layer 21 and the dodecane-soluble resin layer 23. The skin-adhesive film 10C has a layer including the attachment surface. The layer may be either the polylactic acid layer 21 or the dodecane-soluble resin layer 23. The resistance of the dodecane-soluble resin layer 23 to some cosmetics and water is higher than that of the polylactic acid layer 21. Therefore, it is preferable that the one of the attachment surface and the surface facing to the attachment surface of the skin-adhesive film 10C that comes into contact with the cosmetics or water is the surface that has the dodecane-soluble resin layer 23.

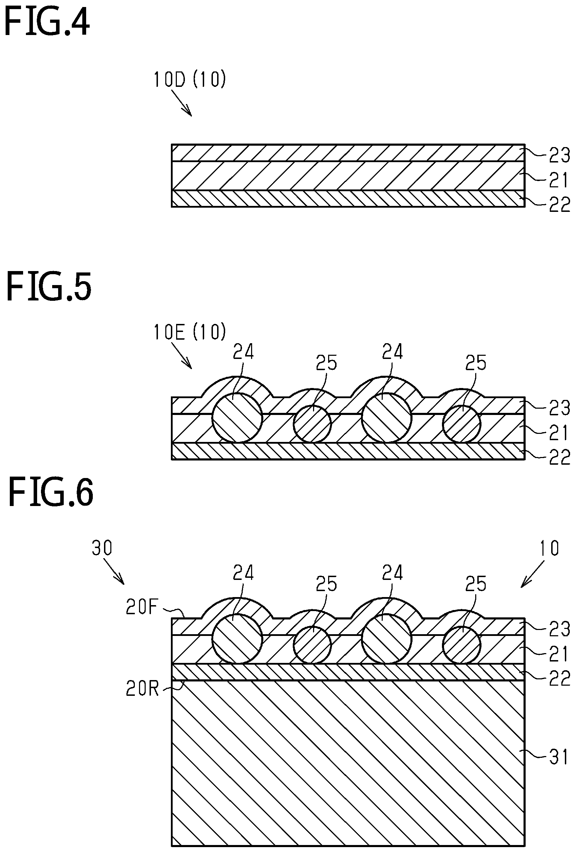

Fig. 4 shows the structure of the skin-adhesive film 10D according to a fourth mode. The skin-adhesive film 10D includes the polylactic acid layer 21, the water-soluble resin layer 22, and the dodecane-soluble resin layer 23. The polylactic acid layer 21 is sandwiched between the water-soluble resin layer 22 and the dodecane-soluble resin layer 23. In other words, one of the two surfaces of the polylactic acid layer 21 is covered by the water-soluble resin layer 22, and the other is covered by the dodecane-soluble resin layer 23.

The skin-adhesive film 10D of the fourth mode has a three-layer structure including the polylactic acid layer 21, the water-soluble resin layer 22, and the dodecane-soluble resin layer 23. The skin-adhesive film 10D has a layer including the attachment surface. The layer may be either the water-soluble resin layer 22 or the dodecane-soluble resin layer 23. The layer having the attachment surface can be selected according to the types of the cosmetics or the like with which the attachment surface and the surface facing to the attachment surface of the skin-adhesive film 10D come into contact.

As described above, if the water-soluble resin layer 22 has the attachment surface, the water-soluble resin layer 22 can provide the effect of enhancing the adhesion between the skin and the skin-adhesive film 10B. Further, the dodecane-soluble resin layer 23 can enhance the resistance of the skin-adhesive film 10C to water in addition to the resistance of the film 10C to cosmetics. To take advantage of these characteristics, it is preferable that the water-soluble resin layer 22 has the attachment surface and the dodecane-soluble resin layer 23 has the outermost surface facing to the attachment surface.

Fig. 5 shows the structure of the skin-adhesive film 10E according to a fifth mode. The skin-adhesive film 10E includes the polylactic acid layer 21, the water-soluble resin layer 22, and the dodecane-soluble resin layer 23, and the polylactic acid layer 21 is sandwiched between the water-soluble resin layer 22 and the dodecane-soluble resin layer 23. Further, the polylactic acid layer 21 contains water-soluble resin particles 24 and dodecane-soluble resin particles 25.

Each of the water-soluble resin particles 24 and the dodecane-soluble resin particles 25 has a particle diameter that is larger than the thickness of the part of the polylactic acid layer 21 that does not contain these particles 24 and 25. That is, each of the water-soluble resin particles 24 and the dodecane-soluble resin particles 25 are exposed at the surface of the polylactic acid layer 21.

The water-soluble resin particles 24 are made of water-soluble resin, and they serve to enhance the adhesion between the polylactic acid layer 21 and the water-soluble resin layer 22. The dodecane-soluble resin particles 25 are made of dodecane-soluble resin, and they serve to enhance the adhesion between the polylactic acid layer 21 and the dodecane-soluble resin layer 23.

The skin-adhesive film 10E of the fifth mode has a three-layer structure including the polylactic acid layer 21, the water-soluble resin layer 22, and the dodecane-soluble resin layer 23. The skin-adhesive film 10E has a layer including the attachment surface. The layer may be either the water-soluble resin layer 22 or the dodecane-soluble resin layer 23.

Fig. 5 shows an example where the water-soluble resin layer 22 is a flat layer and the dodecane-soluble resin layer 23 has a film form conforming to unevenness of the polylactic acid layer 21 due to the water-soluble resin particles 24 and the dodecane-soluble resin particles 25. The structure is not limited to this, and it is possible that the dodecane-soluble resin layer 23 may be a flat layer and the water-soluble resin layer 22 may have a film form conforming to the unevenness of the polylactic acid layer 21 due to the particles 24 and 25. Alternatively, both of the water-soluble resin layer 22 and the dodecane-soluble resin layer 23 may have a film form conforming to the unevenness of the polylactic acid layer 21 due to the particles 24 and 25. The layer having unevenness may be determined based on the order in which layers are formed, the magnitude of the pressure applied in the manufacturing process, and the like.

As described above, to take advantage of the characteristics of the water-soluble resin layer 22 and the dodecane-soluble resin layer 23, it is preferable that the water-soluble resin layer 22 has the attachment surface and the dodecane-soluble resin layer 23 has the outermost surface facing to the attachment surface. Further, when the outermost surface facing to the attachment surface has unevenness, it is possible to prevent the skin from appearing glossy, in other words, greasy or shiny, in an area where the skin-adhesive film 10E is attached. In addition, when cosmetics such as foundation are applied over the skin-adhesive film 10E, the film 10E helps the cosmetics to be retained on the film 10E. As the cosmetics stay on well, in other words, the cosmetics settle and sit well, the makeup dose not easily come off. Therefore, when the dodecane-soluble resin layer 23 has the outermost surface, it is preferable that at least the dodecane-soluble resin layer 23 has the unevenness conforming to the particles 24 and 25.

Fig. 6 shows the structure of a transfer sheet 30. The transfer sheet 30 includes the skin-adhesive film 10 and a support substrate 31 supporting the film 10. The support substrate 31 is in contact with a support surface 20R which is the outermost surface of the skin-adhesive film 10. The support surface 20R and an attachment surface 20F are opposite side surfaces of the skin-adhesive film 10.

The support substrate 31 is configured to be peelable off from the skin-adhesive film 10, and the support substrate 31 is peeled off from the film 10 after the film 10 is attached to the skin. Since the skin-adhesive film 10 is supported by the support substrate 31, the film 10 can easily be attached to the skin.

Although Fig. 6 shows the case where the transfer sheet 30 includes the skin-adhesive film 10E of the fifth mode, the film 10 included in the transfer sheet 30 may be any of the film 10 in first to fifth modes.

### [Material of Skin-Adhesive Film]

In the above-described modes, the skin-adhesive film 10 has a thickness of 10 nm or more and 5000 nm or less. The thickness of the skin-adhesive film 10 and the thickness of each layer according to the present embodiment are an average thickness obtained by measuring the film thickness at five or more measuring points by observing the cross-section using an electron microscope. When the thickness of the skin-adhesive film 10 is 5000 nm or less, the film 10 is thin enough for the film 10 to conform to the skin texture, that is, the unevenness of the skin. Therefore, the distance between the skin and the skin-adhesive film 10 is reduced, and the van der Waals force that acts between them becomes larger. As a result, sufficient adhesion is obtained between the skin and the skin-adhesive film 10 without using an adhesive or the like. In order to further increase the adhesion between the skin and the skin-adhesive film 10, the thickness of the film 10 is preferably 1000 nm or less.

When the thickness of the skin-adhesive film 10 is 10 nm or more, the film 10 may obtain strength enough to be used practically. Thus, it is possible to prevent the film 10 from being torn extremely easily when handling it. In order to further increase the strength of the skin-adhesive film 10, the thickness of the film 10 is preferably 100 nm or more.

A material of the skin-adhesive film 10 will be described. It is desirable that the skin-adhesive film 10 is made of a material that is safe for a living body because it is attached to the skin. Examples of such materials include polyester, cellulosic-based, and silicone-based materials. In particular, since poly-L-lactic acid has been used as a material for medical products applied to a living body, such as adhesion barrier membranes and sutures, it can be said that it is a preferable material in terms of biocompatibility.

However, as described above, it is difficult to use melt extrusion to form an extremely thin film, and thus solvent casting is used. Since poly-L-lactic acid dissolves only in a halogen-based solvent, a halogen-based solvent needs to be used to form a thin film made of poly-L-lactic acid, which causes a safety problem.

Poly-L-lactic acid dissolves only in a halogen-based solvent because poly-L-lactic acid is crystalline. On the other hand, poly-DL-lactic acid, which is a random copolymer of L-lactic acid and D-lactic acid, is amorphous and can be dissolved in an ester-based solvent such as ethyl acetate or n-butyl acetate. In view of this, in the present embodiment, poly-DL-lactic acid is used as the polylactic acid constituting the polylactic acid layer 21.

Ester-based solvents are safer than halogen-based solvents. In particular, ethyl acetate and n-butyl acetate have been designated as food additives in Japan, and are safer to the human body than dichloromethane and chloroform. When the ratio of L-lactic acid in poly-DL-lactic acid is 10% or more and 90% or less, the poly-DL-lactic acid is soluble in ethyl acetate and n-butyl acetate.

Comparing the safety of L-lactic acid to that of D-lactic acid, it is reported that the metabolism of D-lactic acid is worse than that of L-lactic acid in infants (Droese & Stolley, 1965). Therefore, in order to further enhance safety to the human body, it is preferable that L-lactic acid is present in higher proportion than D-lactic acid in the poly-DL-lactic acid. Accordingly, in the present embodiment, a poly-DL-lactic acid including L-lactic acid having a content rate of 50% or higher and 90% or lower is used as the polylactic acid constituting the polylactic acid layer 21. The higher the ratio of L-lactic acid in poly-DL-lactic acid is, the higher the resistance of the polylactic acid layer 21 to chemicals, that is, the resistance to cosmetics is.

Although poly-DL-lactic acid is soluble in ester-based solvents, it has low resistance to alcohol-based solvents and hydrocarbon-based solvents contained in cosmetics, oils serving as moisturizing components, ultraviolet absorbers, and the like. Thus, a thin film made of poly-DL-lactic acid is more easily twisted or become torn when the film comes in contact with cosmetics as compared with a thin film made of poly-L-lactic acid. To address such problems, in the present embodiment, a poly-DL-lactic acid having a weight-average molecular weight of 100,000 or higher is used as the poly-DL-lactic acid forming the polylactic acid layer 21. If the weight-average molecular weight of poly-DL-lactic acid is 100,000 or higher, the resistance of the polylactic acid layer 21 to some frequently used cosmetics such as milky lotion and cosmetic solution would be good enough not to cause any problems in use. If the weight-average molecular weight of poly-DL-lactic acid is 100,000 or higher, the film strength of the polylactic acid layer 21 would be suitable for a skin-adhesive film. Thus, it is possible to prevent the polylactic acid layer 21 from being twisted or become torn when the polylactic acid layer 21 is brought into contact with cosmetics.

Further, the weight-average molecular weight of the poly-DL-lactic acid constituting the polylactic acid layer 21 is preferably 200,000 or higher. If the weight-average molecular weight of the poly-DL-lactic acid is 200,000 or higher, the above-described resistance of the polylactic acid layer 21 to cosmetics and the film strength is further increased. The weight-average molecular weight of the poly-DL-lactic acid constituting the polylactic acid layer 21 is preferably lower than 800,000. The polymerization reaction of poly-DL-lactic acid is an esterification reaction, which is an equilibrium reaction involving not only polymerization but also decomposition. If a large amount of impurities such as water and lactic acid is mixed in the intermediate lactide which serves as a raw material of the polymer, it is difficult to produce a polymer having a large weight-average molecular weight. In other words, to produce a polymer having a large weight-average molecular weight, it is necessary to prepare lactide with high purity, which increases production cost. If the weight-average molecular weight of poly-DL-lactic acid is lower than 800,000, because the weight-average molecular weight is not too large, the increase in the production cost is suppressed, and the productivity of the skin-adhesive film 10 can be improved.

In general, when polylactic acid is used as a material of medical products to be placed in the body, polylactic acid having a weight-average molecular weight of 10,000 or higher and 50,000 or lower is often used in order to ensure that the polylactic acid is decomposed in the body and then excreted.

The values of weight-average molecular weight specified in the present embodiment indicate the values of weight average molecular weight measured by gel permeation chromatography (GPC). That is, they are polystyrene-equivalent values measured by dissolving polylactic acid in chloroform and performing gel permeation chromatography.

The poly-DL-lactic acid is produced by known methods. In general, two molecules of lactic acid are dehydrated to produce lactide, which is a cyclic ester, and polylactic acid is produced by ring-opening polymerization of the lactide. If only L-lactic acid is used as the starting material, crystalline poly-L-lactic acid is produced, and if L-lactic acid and D-lactic acid are used, amorphous poly-DL-lactic acid is produced. An example of the polymerization catalyst is tin octylate. Alcohol may also be used to adjust the molecular weight.

The polylactic acid layer 21 may contain other components in addition to poly-DL-lactic acid. Examples of the other components include a component that serves as a plasticizer such as oil, a resin component that is compatible with polylactic acid for improving film strength, particles such as fillers, and the like. Examples of the particles contained in the polylactic acid layer 21 include inorganic fillers such as silica particles, mica particles, alumina particles, barium sulfate particles, calcium carbonate particles, and organic fillers such as polyethylene particles, polypropylene particles, and tetrafluoroethylene particles. The volume mean diameter of the particles contained in the polylactic acid layer 21 measured by the laser diffraction scattering method may be, for example, 50 nm or larger and 10 µm or smaller. In view of suppressing particle aggregation in the step of forming the polylactic acid layer 21 by solvent casting, the volume mean diameter is preferably 500 nm or larger. Also, in view of suppressing particle sedimentation in the same forming step, it is preferably 5 µm or smaller.

In the present embodiment, the main component of the polylactic acid layer 21 refers to a substance that has the highest content (mass%) among the substances constituting the polylactic acid layer 21. The content of poly-DL-lactic acid in the polylactic acid layer 21 is preferably 60% by mass or more.

The polylactic acid layer 21 has a thickness of 10 nm or more and 5000 nm or less. To enhance the adhesion to the skin, the thickness of the portion of the polylactic acid layer 21 that does not contain particles is preferably 1000 nm or smaller, and more preferably 400 nm or smaller. The polylactic acid layer 21 is formed by solvent casting. That is, after dissolving the material of the polylactic acid layer 21 containing poly-DL-lactic acid in a solvent, the solution is applied to a substrate, and the coating film is dried to solidify the film. After that, the solidified film is peeled off from the substrate to obtain the polylactic acid layer 21 which is a thin film. Examples of the method of applying the solution to the substrate include known coating methods such as direct gravure, reverse gravure, small-diameter reverse gravure, Mayer coating, die coating, curtain coating, spraying, spin coating, screen printing, comma coating, knife coating, gravure offset, roll coating, and the like. The thickness of the polylactic acid layer 21 is controlled by the proportion of the solid content in the solution and the coating method.

As described above, using poly-DL-lactic acid having a weight-average molecular weight of 100,000 or higher as the main component of the polylactic acid layer 21, the film obtains good resistance to some cosmetics. However, since sun oil and sunscreen, for example, often contain a component that dissolves amorphous polylactic acid, it cannot be said a skin-adhesive film 10 composed of the polylactic acid layer 21 only has high versatility. In case the skin-adhesive film 10 is sold in combination with the above-specified cosmetics, even the first mode of the skin-adhesive film 10A composed of the polylactic acid layer 21 only can exert sufficient durability for use. However, in order to increase the versatility of the skin-adhesive film 10, it is preferable that the film 10 has resistance to a wide variety of cosmetics, and preferably it can be used for a long time during daytime, especially, for about 8 hours.

In the second to fifth modes of the skin-adhesive film 10, the polylactic acid layer 21 is protected by the water-soluble resin layer 22 and the dodecane-soluble resin layer 23 so as to increase the resistance of the film 10 to cosmetics as well as the film strength of the film 10. Each of the water-soluble resin layer 22 and the dodecane-soluble resin layer 23 has a thickness smaller than 5.0 µm. Further, each of the water-soluble resin layer 22 and the dodecane-soluble resin layer 23 preferably has a thickness smaller than 1.0 µm.

The resin that is the main component of the water-soluble resin layer 22 is a water-soluble resin, and for example, it may be a polysaccharide such as polyvinyl alcohol, polyvinylpyrrolidone, ethyl cellulose, gelatin, hyaluronic acid, starch, or sacran. The water-soluble resin is poorly soluble in an ester-based solvent. In other words, the water-soluble resin layer 22 is poorly soluble in an ester-based solvent that dissolves poly-DL-lactic acid. Therefore, laminating the water-soluble resin layer 22 on the polylactic acid layer 21 makes it possible to prevent the skin-adhesive film 10 from being twisted or become torn if the film 10 comes into contact with cosmetics that contain components dissolving the polylactic acid layer 21. In the present embodiment, the phrase "poorly soluble in an ester-based solvent that dissolves poly-DL-lactic acid" means that, if 1.0 g of a solute is added to 100 g of an ester-based solvent, and the solvent is heated to a temperature that does not exceed the boiling point at normal pressure and stirred for 24 hours, there is more undissolved solute than the case where the poly-DL-lactic acid constituting the polylactic acid layer 21 is dissolved in the same manner.

The water-soluble resin layer 22 may contain various additives depending on the purpose, for example, a plasticizer such as higher fatty acid, higher fatty acid ester, or higher alcohol, and a filler such as acrylic particles, urethane particles, or silica particles. The volume mean diameter of the particles added to the water-soluble resin layer 22 as measured by the laser diffraction scattering method may be, for example, 50 nm or larger and 10 µm or smaller. In view of suppressing particle aggregation in the step of forming the water-soluble resin layer 22 by solvent casting, the volume mean diameter is preferably 500 nm or larger. Also, in view of suppressing particle sedimentation in the same forming step, it is preferably 5 µm or smaller. In the present embodiment, the main component of the water-soluble resin layer 22 refers to the substance that has the highest content (mass%) among the substances constituting the water-soluble resin layer 22.

The resin that is the main component of the dodecane-soluble resin layer 23 can be any suitable resin that is soluble in dodecane. Such dodecane-soluble resins are often used as film-forming materials, and also many of them are copolymers with silicone-based resin, olefin-based resin, silicone-based resins, or olefin-based resin. Dodecane-soluble resin is often soluble in hydrocarbon-based solvents other than dodecane, although the solution viscosity after the resin is dissolved is different from that dissolved in dodecane. Hydrocarbon-based solvents are roughly classified into paraffinic hydrocarbons, naphthenic hydrocarbons, and aromatic hydrocarbons, and typical solvents include normal hexane, isododecane, cyclohexane, and toluene.

Since the polarity of dodecane-soluble resin is completely different from that of polylactic acid, dodecane-soluble resin is poorly soluble in an ester-based solvent. That is, the dodecane-soluble resin layer 23 is poorly soluble in an ester-based solvent that dissolves poly-DL-lactic acid. Therefore, laminating the dodecane-soluble resin layer 23 on the polylactic acid layer 21 makes it possible to prevent the skin-adhesive film 10 from being twisted or become torn if the film 10 comes into contact with cosmetics that contain components that dissolve the polylactic acid layer 21.

Due to a difference between polarity of the dodecane-soluble resin and the polarity of water, many dodecane-soluble resins do not dissolve or decompose well in water. Producing the dodecane-soluble resin layer 23 from the dodecane-soluble resin which is water-resistant, it is possible to prevent the polylactic acid layer 21 from collapsing due to the polylactic acid being hydrolyzed when the skin-adhesive film 10 comes into contact with water.

The dodecane-soluble resin layer 23 may contain various additives depending on the purpose, for example, a plasticizer such as a higher fatty acid, higher fatty acid ester, or higher alcohol, and a filler such as acrylic particles, urethane particles, or silica particles. The volume mean diameter of the particles added to the dodecane-soluble resin layer 23 as measured by the laser diffraction scattering method may be, for example, 50 nm or larger and 10 µm or smaller. In view of suppressing particle aggregation in the step of forming the dodecane-soluble resin layer 23 by solvent casting, the volume mean diameter is preferably 500 nm or larger. Also, in view of suppressing particle sedimentation in the same forming step, it is preferably 5 µm or smaller. In the present embodiment, the main component of the dodecane-soluble resin layer 23 refers to the substance that has the highest content (mass%) among the substances constituting the dodecane-soluble resin layer 23.

In the second to fifth modes of the skin-adhesive film 10, at least one of the water-soluble resin layer 22 and the dodecane-soluble resin layer 23 covers the polylactic acid layer 21, and therefore the resistance of the film 10 to cosmetics can be enhanced. In addition, as the skin-adhesive film 10 is made of a laminate of thin films, the film strength of the film 10 can be enhanced. If the skin-adhesive film 10 has a high film strength, the film 10 is less likely to get become torn. For example, it is possible to prevent the skin-adhesive film 10 from being become torn when it is attached to the skin, and it is also possible to prevent the film 10 from being become torn when the surface of the film 10 is rubbed due to contact with the user's hand or the like after the film 10 is attached to the skin. Since the durability of the skin-adhesive film 10 is improved, the skin-adhesive film 10 can be used suitably for a long-time.

In the fourth and fifth modes of the skin-adhesive film 10, the polylactic acid layer 21 is sandwiched between the water-soluble resin layer 22 and the dodecane-soluble resin layer 23. Thus, the skin-adhesive film 10 has higher resistance to more kinds of cosmetics.

Due to a difference in polarity between the water-soluble resin layer 22 and the polylactic acid layer 21, adhesion between the water-soluble resin layer 22 and the polylactic acid layer 21 may decrease. Similarly, due to a difference in polarity between the dodecane-soluble resin layer 23 and the polylactic acid layer 21, the adhesion between the dodecane-soluble resin layer 23 and the polylactic acid layer 21 may decrease. To improve the adhesion between layers, the polylactic acid layer 21 of the skin-adhesive film 10E in the fifth mode contains the water-soluble resin particles 24 and the dodecane-soluble resin particles 25.

Since the water-soluble resin particles 24 are exposed at the surface of the polylactic acid layer 21, the water-soluble resin layer 22 covering the polylactic acid layer 21 comes into contact with the water-soluble resin particles 24 at a part where the water-soluble resin particles 24 are located. An affinity of water-soluble resin for water-soluble resin is higher than an affinity of water-soluble resin for polylactic acid. Thus, if the polylactic acid layer 21 contains the water-soluble resin particles 24, as compared with the case where the entire surface of the water-soluble resin layer 22 comes into contact with a part made of polylactic acid, the adhesion between the water-soluble resin layer 22 and the polylactic acid layer 21 can be enhanced.

Similarly, since the dodecane-soluble resin particles 25 are exposed at the surface of the polylactic acid layer 21, the dodecane-soluble resin layer 23 covering the polylactic acid layer 21 comes into contact with the dodecane-soluble resin particles 25 at a part where the dodecane-soluble resin particles 25 are located. An affinity of dodecane-soluble resin for dodecane-soluble resin is higher than an affinity of dodecane-soluble resin for polylactic acid. Thus, if the polylactic acid layer 21 contains the dodecane-soluble resin particles 25, as compared with the case where the entire surface of the dodecane-soluble resin layer 23 comes into contact with a part composed of polylactic acid, the adhesion between the dodecane-soluble resin layer 23 and the polylactic acid layer 21 is enhanced.

The water-soluble resin constituting the water-soluble resin particles 24 may be, for example, one of the resins exemplified as the material of the water-soluble resin layer 22. The water-soluble resin particles 24 and the water-soluble resin layer 22 may be made of the same water-soluble resin, or may be made of different water-soluble resins. In order to further enhance adhesion between the water-soluble resin layer 22 and the polylactic acid layer 21, preferably the water-soluble resin constituting the water-soluble resin particles 24 matches the water-soluble resin constituting the water-soluble resin layer 22. The volume mean diameter of the water-soluble resin particles 24 contained in the polylactic acid layer 21 which is measured by the laser diffraction scattering may be, for example, 100 nm or larger and 10 µm or smaller. In view of suppressing particle aggregation in the step of forming the polylactic acid layer 21 by solvent casting, the volume mean diameter is preferably 500 nm or larger. Also, in view of suppressing particle sedimentation in the same forming step, it is preferably 5 µm or smaller.

The dodecane-soluble resin constituting the dodecane-soluble resin particles 25 may be, for example, one of the resins exemplified as the material of the dodecane-soluble resin layer 23. The dodecane-soluble resin particles 25 and the dodecane-soluble resin layer 23 may be made of the same dodecane-soluble resin, or may be made of different dodecane-soluble resins. In order to further enhance the adhesion between the dodecane-soluble resin layer 23 and the polylactic acid layer 21, preferably the dodecane-soluble resin constituting the dodecane-soluble resin particles 25 matches the dodecane-soluble resin constituting the dodecane-soluble resin layer 23. In addition, the volume mean diameter of the dodecane-soluble resin particles 25 contained in the polylactic acid layer 21 as measured by the laser diffraction scattering may be, for example, 100 nm or larger and 10 µm or smaller. In view of suppressing particle aggregation in the step of forming the polylactic acid layer 21 by solvent casting, the volume mean diameter is preferably 500 nm or larger. Also, in view of suppressing particle sedimentation in the same forming step, it is preferably 5 µm or smaller.

Next, the support substrate 31 included in the transfer sheet 30 will be described. The support substrate 31 protects the skin-adhesive film 10 by supporting the skin-adhesive film 10. Since the skin-adhesive film 10 is extremely thin, the strength of the film 10 is weaker than that of a general film used for packaging materials or the like. Therefore, since the skin-adhesive film 10 would be handled in a state in which it is supported by the support substrate 31 until it is attached to the skin, it is possible to prevent the film 10 from being damaged or become torn.

For example, preferably, the support substrate 31 may be, for example, a film, a non-woven fabric, a sheet of paper, or the like. Examples of the film may be one composed of polyethylene terephthalate, polyethylene naphthalate, polyethylene, polypropylene, cycloolefin polymer, cycloolefin copolymer, nylon, or the like. The support substrate 31 may be provided with, on the surface of the layer made of such material, a layer for facilitating the support substrate 31 to be peeled off from the skin-adhesive film 10, made of silicone, fluororesin, olefin, wax, a film-forming material, water-soluble resin, or the like. In particular, a support substrate 31 having a layer made of a releasable resin such as silicone or olefin on the surface of the substrate 31, or a support substrate 31 such as a non-woven fabric or a sheet of paper that easily swells due to contact with a liquid such as water so that its release properties from the skin-adhesive film 10 increase, is preferable as they are easily peelable off from the film 10. The skin-adhesive film 10 and the support substrate 31 may be detachably fixed to each other by using a weak tackifier or a weak adhesive. The skin-adhesive film 10 and the support substrate 31 may be heated or pressurized to fix them to each other.

### [Method for Attaching Skin-Adhesive Film]

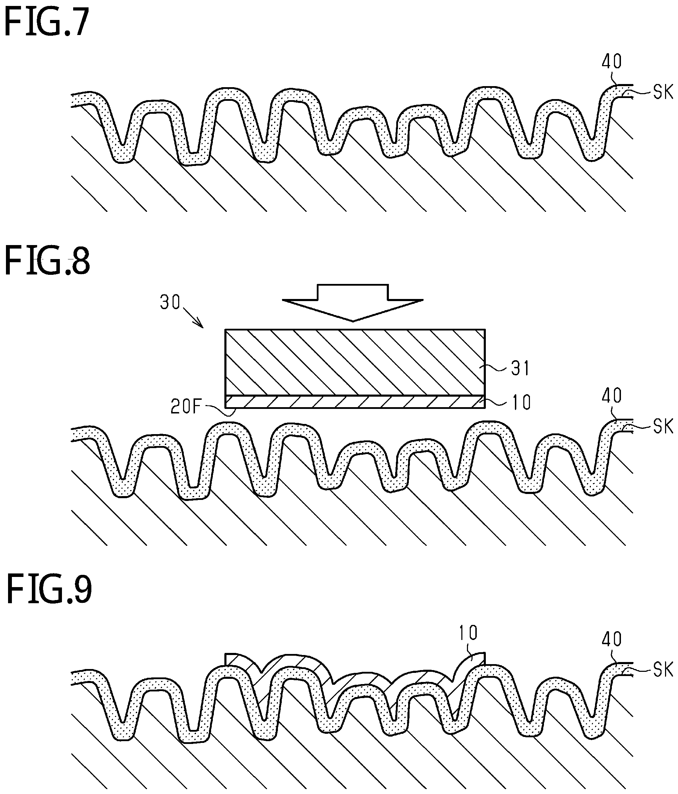

A method of the skin-adhesive film 10 being attached using the transfer sheet 30 will be described with reference to Figs. 7 to 9. In the following, as an example, the transfer sheet 30 provided with the skin-adhesive film 10A of the first mode is used. In this example, description will be given of the case where the skin-adhesive film 10 is attached to skin to which cosmetics have already been applied.

As shown in Fig. 7, first, cosmetics 40 such as skin lotion are applied to a skin SK. Water may be applied instead of the cosmetic 40.

As shown in Fig. 8, the transfer sheet 30 is then pressed against the skin SK so that the attachment surface 20F of the skin-adhesive film 10 comes into contact with the skin SK to which the cosmetic 40 is applied. Then, the support substrate 31 is peeled off from the skin-adhesive film 10.

As a result, as shown in Fig. 9, the skin-adhesive film 10 is transferred to the skin SK from the transfer sheet 30, and the film 10 is attached to the skin SK. Since the skin-adhesive film 10 is extremely thin, it conforms to the unevenness of the skin SK and adheres well to the skin SK. The skin-adhesive film 10 has enough adhesion to the skin SK. The adhesion strength is of a degree at which it does not peel off naturally.

Since evaporation of sebum and sweat is suppressed by covering the skin SK with the skin-adhesive film 10, a moisturizing effect can be obtained. Further, if a cosmetic such as foundation is applied over the skin-adhesive film 10, the cosmetic stays on better than in the case where it is directly applied to the skin. In addition, it is possible to prevent the skin SK from coming into contact with harmful bacteria or substances. Thus, the skin-adhesive film 10 functions as an auxiliary material for skin care or a makeup foundation.

### [Examples]

The skin-adhesive film and the transfer sheet will be described by way of specific examples and comparative examples.

### (Example 1)

### <Purification of Polylactic Acid>

Polylactic acid was produced by ring-opening polymerization of lactide. Specifically, L-lactide and D-lactide were polymerized by mixing them at a weight ratio of L-lactide: D-lactide = 50:50 using tin octylate as a catalyst and lauryl alcohol as a polymerization initiator and molecular weight modifier. The amount of unreacted lactide was measured by gas chromatography, and the content of lactide in the product was 5%. The product was purified by recrystallization to lower the unreacted lactide content to 0.1%. The obtained polylactic acid was dissolved in chloroform and its molecular weight was measured by GPC. The weight-average molecular weight was 100,000.

### <Preparation of Transfer Sheet>

The poly-DL-lactic acid (proportion of L-lactic acid: 50%) purified as described above was heated to 60°C and dissolved in ethyl acetate. The obtained solution was applied to a release substrate using microgravure coating, and the coating film was dried to form a polylactic acid layer. The thickness after drying was 200 nm. A non-woven fabric was laminated as a support substrate on the polylactic acid layer on the release substrate, and the release substrate was peeled off from an edge part. The transfer sheet in Example 1, in which a skin-adhesive film solely composed of the polylactic acid layer is supported by the support substrate, was thus obtained.

### (Example 2)

### <Purification of Polylactic Acid>

Polylactic acid was produced by ring-opening polymerization of lactide. First, L-lactide and D-lactide were purified by recrystallization in order to produce a polylactic acid having a large weight-average molecular weight. The L-lactide and D-lactide thus obtained were polymerized by mixing them at a weight ratio of L-lactide: D-lactide = 50:50 using tin octylate as the catalyst and lauryl alcohol as the polymerization initiator and molecular weight modifier. The amount of unreacted lactide was measured by gas chromatography, and the content of lactide in the product was 5%. The obtained polylactic acid was dissolved in chloroform and its molecular weight was measured by GPC. The weight-average molecular weight was 400,000.

### <Preparation of Transfer Sheet>

The polylactic acid layer was formed in the same manner as in Example 1 using the poly-DL-lactic acid (proportion of L-lactic acid: 50%) purified as described above, and the transfer sheet was formed in the same manner as in Example 1. The transfer sheet in Example 2, in which the skin-adhesive film solely composed of the polylactic acid layer is supported by the support substrate, was thus obtained.

### (Example 3)

### <Purification of Polylactic Acid>

Polylactic acid was produced by ring-opening polymerization of lactide. Specifically, L-lactide and D-lactide were polymerized by mixing them at a weight ratio of L-lactide: D-lactide = 90:10 using tin octylate as the catalyst and lauryl alcohol as the polymerization initiator and molecular weight modifier. The amount of unreacted lactide was measured by gas chromatography, and the content of lactide in the product was 5%. The obtained polylactic acid was dissolved in chloroform and its molecular weight was measured by GPC. The weight-average molecular weight was 100,000.

### <Preparation of Transfer Sheet>

The polylactic acid layer was formed in the same manner as in Example 1 using the poly-DL-lactic acid (proportion of L-lactic acid: 90%) purified as described above, and the transfer sheet was formed in the same manner as in Example 1. The transfer sheet in Example 3, in which the skin-adhesive film solely composed of the polylactic acid layer is supported by the support substrate, was thus obtained.

### (Example 4)

### <Purification of Polylactic Acid>

Polylactic acid was produced by ring-opening polymerization of lactide. Specifically, L-lactide and D-lactide were polymerized by mixing them at a weight ratio of L-lactide: D-lactide = 80: 20 using tin octylate as the catalyst and lauryl alcohol as the polymerization initiator and molecular weight modifier. The amount of unreacted lactide was measured by gas chromatography, and the content of lactide in the product was 5%. The obtained polylactic acid was dissolved in chloroform and its molecular weight was measured by GPC. The weight-average molecular weight was 200,000.

### <Preparation of Transfer Sheet>

The polylactic acid layer was formed in the same manner as in Example 1 using the poly-DL-lactic acid (proportion of L-lactic acid: 80%) purified as described above, and the transfer sheet was formed in the same manner as in Example 1. The transfer sheet in Example 4, in which a skin-adhesive film solely composed of the polylactic acid layer is supported by the support substrate, was thus obtained.

### (Example 5)

### <Purification of Polylactic Acid>

Polylactic acid was produced by ring-opening polymerization of lactide. Specifically, L-lactide and D-lactide were polymerized by mixing them at a weight ratio of L-lactide: D-lactide = 50:50 using tin octylate as a catalyst and lauryl alcohol as a polymerization initiator and molecular weight modifier. The amount of unreacted lactide was measured by gas chromatography, and the content of lactide in the product was 5%. The obtained polylactic acid was dissolved in chloroform and its molecular weight was measured by GPC. The weight-average molecular weight was 200,000.

### <Preparation of Transfer Sheet>

Sacran, which is an extract from Aphanothece sacrum, was dissolved in water, and the obtained solution was applied to a release substrate using microgravure coating. The coating film was dried to form a water-soluble resin layer. The thickness after drying was 400 nm.

After that, the poly-DL-lactic acid (proportion of L-lactic acid: 50%) purified as described above was heated to 60°C and dissolved in ethyl acetate, and the obtained solution was applied to the water-soluble resin layer using microgravure coating. The coating film was dried to form a polylactic acid layer. The thickness after drying was 400 nm. A skin-adhesive film with a thickness of 800 nm was thus formed. A non-woven fabric was laminated as a support substrate on the polylactic acid layer on the release substrate, and the release substrate was peeled off from an edge part. The transfer sheet in Example 5, in which a skin-adhesive film including the polylactic acid layer and the water-soluble resin layer is supported by the support substrate, was thus obtained.

### (Example 6)

### <Purification of Polylactic Acid>

Polylactic acid was produced by ring-opening polymerization of lactide. Specifically, L-lactide and D-lactide were polymerized by mixing them at a weight ratio of L-lactide: D-lactide = 50:50 using tin octylate as a catalyst and lauryl alcohol as a polymerization initiator and molecular weight modifier. The amount of unreacted lactide was measured by gas chromatography, and the content of lactide in the product was 5%. The obtained polylactic acid was dissolved in chloroform and its molecular weight was measured by GPC. The weight-average molecular weight was 200,000.

### <Preparation of Water-Soluble Resin Particles and Dodecane-Soluble Resin Particles>

Water-soluble resin particles are obtained by polyvinyl alcohol being dissolved in water, crystallized using a mixed solvent of isopropyl alcohol and ethyl acetate, and the precipitate being filtered and dried. Further, a (acrylate/polytrimethylsiloxy methacrylate) copolymer (manufactured by Toray Dow Corning Co.) dissolved in isododecane was crystallized using an ethyl acetate solvent, and the precipitate was filtered and dried to obtain dodecane-soluble resin particles.

### <Preparation of Transfer Sheet>

Sacran was dissolved in water and the obtained solution was applied to the release substrate using microgravure coating. The coating film was dried to form a water-soluble resin layer. A thickness after drying was 200 nm.

Next, the poly-DL-lactic acid (proportion of L-lactic acid: 50%) purified as described above was heated to 60°C and dissolved in ethyl acetate to obtain a solution. The water-soluble resin particles and the dodecane-soluble resin particles were added to the obtained polylactic acid solution respectively at a ratio of 1.0 mass% and stirred to obtain a coating solution. The coating solution was applied to the water-soluble resin layer using microgravure coating. The coating film was dried to form a polylactic acid layer. A thickness after drying was 300 nm.

Then, a solution of a (acrylate/polytrimethylsiloxy methacrylate) copolymer (manufactured by Toray Dow Corning Co.) dissolved in isododecane was applied to the polylactic acid layer using microgravure coating. The coating film was dried to form a dodecane-soluble resin layer. The thickness after drying was 200 nm.

A skin-adhesive film with a thickness of 700 nm was thus formed. A non-woven fabric was laminated as a support substrate on the dodecane-soluble resin layer on the release substrate, and the release substrate was peeled off from an end part. The transfer sheet in Example 6, in which a skin-adhesive film including the polylactic acid layer, the water-soluble resin layer, and the dodecane-soluble resin layer is supported by the support substrate, was thus obtained.

### (Comparative Example 1)

### <Purification of Polylactic Acid>

Polylactic acid was produced by ring-opening polymerization of lactide. Specifically, L-lactide and D-lactide were polymerized by mixing them at a weight ratio of L-lactide: D-lactide = 95: 5 using tin octylate as the catalyst and lauryl alcohol as the polymerization initiator and molecular weight modifier. The amount of unreacted lactide was measured by gas chromatography, and the content of lactide in the product was 5%. The obtained polylactic acid was dissolved in chloroform and its molecular weight was measured by GPC. The weight-average molecular weight was 100,000.

### <Preparation of Transfer Sheet>

The poly-DL-lactic acid (proportion of L-lactic acid: 95%) purified as described above was heated to 60°C. Then, the inventors attempted to dissolve it in ethyl acetate. However, the poly-DL-lactic acid did not dissolve in ethyl acetate. Therefore, no skin-adhesive film or transfer sheet could be formed in Comparative Example 1.

### (Comparative Example 2)

### <Purification of Polylactic Acid>

Polylactic acid was produced by ring-opening polymerization of lactide. Specifically, L-lactide and D-lactide were polymerized by mixing them at a weight ratio of L-lactide: D-lactide = 50:50 using tin octylate as a catalyst and lauryl alcohol as a polymerization initiator and molecular weight modifier. The amount of unreacted lactide was measured by gas chromatography, and the content of lactide in the product was 5%. The product was purified by recrystallization to lower the unreacted lactide content to 0.1%. The obtained polylactic acid was dissolved in chloroform and its molecular weight was measured by GPC. The weight-average molecular weight was 50,000.

### <Preparation of Transfer Sheet>

The polylactic acid layer was formed in the same manner as in Example 1 using the poly-DL-lactic acid (proportion of L-lactic acid: 50%) purified as described above, and the transfer sheet was formed in the same manner as in Example 1. The transfer sheet in Comparative Example 2, in which a skin-adhesive film solely composed of the polylactic acid layer is supported by the support substrate, was thus obtained.

### (Evaluation Method)

### <Solubility in Solvent>

Each of the poly-DL-lactic acids prepared for the Examples and the Comparative Examples was dissolved in ethyl acetate, and their solubilities were evaluated. 100 g of ethyl acetate was put into a container with 10 g of poly-DL-lactic acid, and they were caused to spin together in the container at 100 revolutions per minute. The poly-DL-lactic acid samples were evaluated as having "high solubility" if they dissolved completely within an hour. Further, the poly-DL-lactic acid samples were evaluated as having "moderate solubility" if they dissolved completely by at least one of heating at 60°C or stirring for a longtime (within 24 hours), and evaluated as having "low solubility" if they did not dissolve completely even by heating at 60°C and stirring for a longtime (within 24 hours). High solubility is represented by "A", moderate solubility is represented by "B", and low solubility is represented by "C".

### <Resistance to Cosmetics>

After dripping a skin lotion on the surface of artificial leather (SUPPLALE, manufactured by IDEATEX JAPAN Co.,Ltd.), a surface of the artificial leather and the attachment surface of the skin-adhesive film of each of the transfer sheets in the Examples and Comparative Example 2 were attached to each other. Then, only the support substrate was peeled off to transfer the skin-adhesive film onto the artificial leather. After that, a cotton swab was impregnated with a sunscreen containing liquid ultraviolet absorber (ethylhexyl methoxycinnamate), and a rubbing test was performed by rubbing the upper surface of the skin-adhesive film with the cotton swab 10 times. The films were visually observed after the rubbing test, and were evaluated as "A" if no tearing was confirmed, "B" if there was an inconspicuous tearing in a very small area, and "C" if tearing was notable.

### <Film Strength>

A strip-shaped test piece having a width of 15 mm was formed from the skin-adhesive film of each of the Examples and Comparative Example 2. A tensile test was performed using a table-top compression/tensile tester (EZ-Test manufactured by Shimadzu Corporation), and the load at which the test piece broke was measured. If the measured value, i.e., the film strength is 30 mN/15 mm or larger, it can be said that the film strength is good enough for a skin-adhesive film. Accordingly, cases where the film strength is less than 30 mN/15 mm were defined as having insufficient film strength, and the samples were evaluated as "A" if the film strength was 100 mN/15 mm or less, "B" if the film strength was 30 mN/15 mm or more and less than 100 mN/15 mm, and "C" if the film strength was less than 30 mN/15 mm.

### (Evaluation Results)

Table 1 shows, for each of the Examples and Comparative Examples, the structure of the skin-adhesive film, the content rate of L-lactic acid in poly-DL-lactic acid, the weight-average molecular weight of poly-DL-lactic acid, and the evaluation results according to the above evaluation methods.

**[Table 1]**

| | Film structure | Content of L-lactic acid (%) | Weight-average molecular weight | Solubility of solvent | Resistance to cosmetics | Film strength |
|---|---|---|---|---|---|---|
| Example 1 | Polylactic acid layer | 50 | 1x10⁵ | A | B | B |
| Example 2 | Polylactic acid layer | 50 | 4x10⁵ | B | A | A |
| Example 3 | Polylactic acid layer | 90 | 1x10⁵ | B | A | B |
| Example 4 | Polylactic acid layer | 80 | 2x10⁵ | A | B | B |
| Example 5 | Water-soluble resin layer Polylactic acid layer | 50 | 2x10⁵ | B | A | A |
| Example 6 | Water-soluble resin layer Polylactic acid layer Dodecane-soluble resin layer + Particles | 50 | 2x10⁵ | B | A | A |
| Comparative Ex. 1 | - | 95 | 1x10⁵ | c | - | - |
| Comparative Ex. 2 | Polylactic acid layer | 50 | 5x10⁴ | A | c | c |

As shown in Table 1, Examples 1 to 6 and Comparative Example 2, in which the content rate of L-lactic acid in poly-DL-lactic acid is 90% or lower, showed good solubility of poly-DL-lactic acid in ethyl acetate. On the other hand, with regard to Comparative Example 1 in which the content rate of L-lactic acid is higher than 90%, poly-DL-lactic acid did not dissolve in ethyl acetate. These results indicate that if poly-DL-lactic acid having an L-lactic acid whose content ratio of 90% or lower is used, the polylactic acid layer can be formed using an ester solvent which is safer than a halogen solvent.

Further, among the Examples and Comparative Examples having a skin-adhesive film composed of only the polylactic acid layer, Examples 1 to 4, in which the weight-average molecular weight of poly-DL-lactic acid is 100,000 or higher, have good resistance to cosmetics and good film strength. On the other hand, with regard to Comparative Example 2 where the weight-average molecular weight of poly-DL-lactic acid is lower than 100,000, the resistance to cosmetics and film strength were both insufficient. Thus, it can be said that a polylactic acid layer having higher resistance to cosmetics can be obtained by using a poly-DL-lactic acid having a weight-average molecular weight of 100,000 or higher.

Comparing Example 1 with Example 2, it may suggest that the larger the weight-average molecular weight of poly-DL-lactic acid is, the better the resistance to cosmetics is. Comparing Example 1 with Example 3, it may suggest that the larger the content rate of L-lactic acid is, the better the resistance to cosmetics is. Comparing Example 1 with Example 2, it may suggest that the larger the weight-average molecular weight of poly-DL-lactic acid is, the better the film strength is.

In addition, as described in Examples 5 and 6, by forming the skin-adhesive film as a laminate of a polylactic acid layer, and a water-soluble resin layer or a dodecane-soluble resin layer, even if a poly-DL-lactic acid having a relatively small weight-average molecular weight is used, the film can be strengthened with regard to both of the resistance to cosmetics and film strength.

As described above in the Embodiments and Examples, according to the skin-adhesive film and the transfer sheet, the effects listed below can be achieved.
(1) The content ratio of L-lactic acid in the poly-DL-lactic acid constituting the polylactic acid layer 21 is 50% or higher and 90% or lower, and the weight-average molecular weight is 100,000 or higher. Since the content ratio of L-lactic acid in the poly-DL-lactic acid is 50% or higher and 90% or lower, the polylactic acid layer 21 can be formed using an ester solvent which is safer than a halogen solvent. Since the weight-average molecular weight of the poly-DL-lactic acid is 100,000 or higher, the resistance of the polylactic acid layer 21 to cosmetics can be enhanced. Therefore, the skin-adhesive film 10 can be manufactured using a solvent safer than a halogen-based solvent, and at the same time a decrease in its resistance to cosmetics is suppressed.
(2) If the weight-average molecular weight of the poly-DL-lactic acid is both 100,000 or higher and lower than 800,000 which is suitable for mass production of poly-DL-lactic acid, productivity in the skin-adhesive film 10 is improved.
(3) If the skin-adhesive film 10 includes the water-soluble resin layer 22, the resistance to cosmetics and film strength of the film 10 is improved.
(4) If the skin-adhesive film 10 includes the dodecane-soluble resin layer 23, the resistance to cosmetics and film strength of the film 10 is increased. In addition, the resistance of the film 10 to water is improved.
(5) If the polylactic acid layer 21 is sandwiched between the water-soluble resin layer 22 and the dodecane-soluble resin layer 23, the skin-adhesive film 10 obtains enhanced resistance to more types of cosmetics.
(6) If the polylactic acid layer 21 contains water-soluble resin particles 24, and the water-soluble resin particles 24 are exposed at the surface of the polylactic acid layer 21 in contact with the water-soluble resin layer 22, the adhesion between the polylactic acid layer 21 and the water-soluble resin layer 22 is enhanced.
(7) If the polylactic acid layer 21 contains dodecane-soluble resin particles 25, and the dodecane-soluble resin particles 25 are exposed at the surface of the polylactic acid layer 21 in contact with the dodecane-soluble resin layer 23, the adhesion between the polylactic acid layer 21 and the dodecane-soluble resin layer 23 is enhanced.
(8) Because the skin-adhesive film 10 of the transfer sheet 30 is supported by the support substrate 31 configured to be peelable off from the skin-adhesive film 10, the film 10 is handled more easily.

### [Modifications]

The above-described embodiments can be implemented with modifications as described below.
- The skin-adhesive film 10B of the second mode which has a two-layer structure of the polylactic acid layer 21 and the water-soluble resin layer 22 may be modified such that the polylactic acid layer 21 includes the water-soluble resin particles 24. According to such configuration, the adhesion between the polylactic acid layer 21 and the water-soluble resin layer 22 is improved.
- The skin-adhesive film 10C in the third mode which has a two-layer structure of the polylactic acid layer 21 and the dodecane-soluble resin layer 23 may be modified such that the polylactic acid layer 21 includes the dodecane-soluble resin particles 25. According to such configuration, the adhesion between the polylactic acid layer 21 and the dodecane-soluble resin layer 23 is improved.
- The skin-adhesive film 10E of the fifth mode may be modified such that the polylactic acid layer 21 contains only one of the water-soluble resin particles 24 and the dodecane-soluble resin particles 25. If the polylactic acid layer 21 contains the water-soluble resin particles 24, the adhesion between the polylactic acid layer 21 and the water-soluble resin layer 22 is improved, and if the polylactic acid layer 21 contains the dodecane-soluble resin particles 25, the adhesion between the polylactic acid layer 21 and the dodecane-soluble resin layer 23 is improved. Comparing the adhesion between the water-soluble resin layer 22 and the dodecane-soluble resin layer 23, the dodecane-soluble resin layer 23 have lower adhesion to polylactic acid. Therefore, if the polylactic acid layer 21 contains only one of the water-soluble resin particles 24 and the dodecane-soluble resin particles 25, the polylactic acid layer 21 preferably contains the dodecane-soluble resin particles 25.
- The skin-adhesive film 10 is composed of at least the polylactic acid layer 21. A configuration of the layers of the film 10 may be different from those of the first to fifth modes. For example, the skin-adhesive film 10 may be configured such that the polylactic acid layer 21 is sandwiched between two water-soluble resin layers 22, or the polylactic acid layer 21 is sandwiched between two dodecane-soluble resin layers 23. Even if the skin-adhesive film 10 is composed of the polylactic acid layer 21, the water-soluble resin layer 22, and dodecane-soluble resin layer 23, they may be laminated in an order different from the order in which the polylactic acid layer 21 is sandwiched between the layer 22 and the layer 23. Alternatively, the skin-adhesive film 10 may include a layer that is different from the water-soluble resin layer 22 and dodecane-soluble resin layer 23.
- The polylactic acid layer 21 may be composed of multiple layers including poly-DL-lactic acid.

### [Reference Signs List]

- 10, 10A, 10B, 10C, 10D, 10E: skin-adhesive film
- 20F: attachment surface
- 20R: Support surface
- 21: Polylactic acid layer
- 22: Water-soluble resin layer
- 23: Dodecane-soluble resin layer
- 24: Water-soluble resin particles
- 25: Dodecane-soluble resin particles
- 30: Transfer sheet
- 31: Support substrate

## Claims

1. A skin-adhesive film including a polylactic acid layer, wherein:
the polylactic acid layer has a thickness of 10 nm or more and 5000 nm or less, the polylactic acid layer has poly-DL-lactic acid, the poly-DL-lactic acid has L-lactic acid having a content ratio of 50% or more and 90% or less, and the poly-DL-lactic acid has a weight-average molecular weight of 100,000 or larger.

2. The skin-adhesive film according to claim 1, wherein:
the weight-average molecular weight of the poly-DL-lactic acid is lower than 800,000.

3. The skin-adhesive film according to claim 1 or 2, further comprising:
a water-soluble resin layer that is poorly soluble in an ester-based solvent that dissolves poly-DL-lactic acid.

4. The skin-adhesive film according to any one of claims 1 to 3, further comprising:
a dodecane-soluble resin layer that is poorly soluble in an ester-based solvent that dissolves poly-DL-lactic acid.

5. The skin-adhesive film according to claim 1 or 2, further comprising:
a water-soluble resin layer that is poorly soluble in an ester-based solvent that dissolves poly-DL-lactic acid; and
a dodecane-soluble resin layer that is poorly soluble in the ester-based solvent, wherein:
the polylactic acid layer is sandwiched between the water-soluble resin layer and the dodecane-soluble resin layer.

6. The skin-adhesive film according to claim 3 or 5, wherein:
the polylactic acid layer contains water-soluble resin particles, and the water-soluble resin particles are exposed at a surface of the polylactic acid layer that comes into contact with the water-soluble resin layer.

7. The skin-adhesive film according to claim 4 or 5, wherein:
the polylactic acid layer contains dodecane-soluble resin particles, and the dodecane-soluble resin particles are exposed at a surface of the polylactic acid layer that comes into contact with the dodecane-soluble resin layer.

8. A transfer sheet comprising:
the skin-adhesive film according to any one of claims 1 to 7; and
a support substrate configured to support the skin-adhesive film and be peelable from the skin-adhesive film.
